# EUROPEAN PATENT SPECIFICATION

(11) **EP 2 018 826 B1**
(45) Date of publication and mention of the grant of the patent: **13.04.2016**
(21) Application number: 08252413.3
(22) Date of filing: 16.07.2008
(51) Int. Cl.: A61B 17/068

(54) **A partially reuseable surgical stapler**
Teilweise wiederverwendbares chirurgisches Klammergerät
Agrafeuse chirurgicale réutilisable partiellement

(30) Priority: 16.07.2007 US 778164
(43) Date of publication of application: 28.01.2009
(73) Proprietor: Ethicon Endo-Surgery, Inc., Cincinnati, OH 45242 (US)
(72) Inventor: Nalagatla, Anil K., Mason, Ohio 45040 (US); Patel, Sudhir B., Mason, Ohio 45040 (US); Pradhan, Debasish, Sambalpur, Orissa 768003 (IN)
(74) Representative: Tunstall, Christopher Stephen

(56) References cited:
- WO-A-03/094745
- US-A- 5 931 847
- US-A1- 2004 108 357
- US-A1- 2007 131 732

## Description

### Field

The present invention relates in general to surgical stapling and also has relation to robotic surgery.

### Background

Before surgical staplers were introduced surgeons had to spend a great deal of time sewing the tissue of patients back together. This was the most time intensive aspect of a surgical procedure. Surgical staplers have decreased the amount of time that a user spends sewing tissue back together. Such surgical staplers are described in the following issued U.S. US4633861 Chow et al.; US4633874 Chow et al.; US5129570 Schulze et al.

One concern is whether the staplers used during procedures are sterile. Reusable staplers, being a relatively complicated mechanical instrument, are difficult to sterilize after use. Hence, it was once desired that the surgical stapler be disposable. As more than one surgical stapler may be required in a surgical procedure, for economical reasons disposable surgical staplers having reloadable staple cartridges have been developed.

Recently, there has been a desire to make a portion of such staples reusable so that a portion is disposable and a portion is reusable.

### Brief Description of the Figures

FIGURE 1 is an Isometric view of the entire device in closed position..
FIGURE 2 is a Front View of the Device in open position.

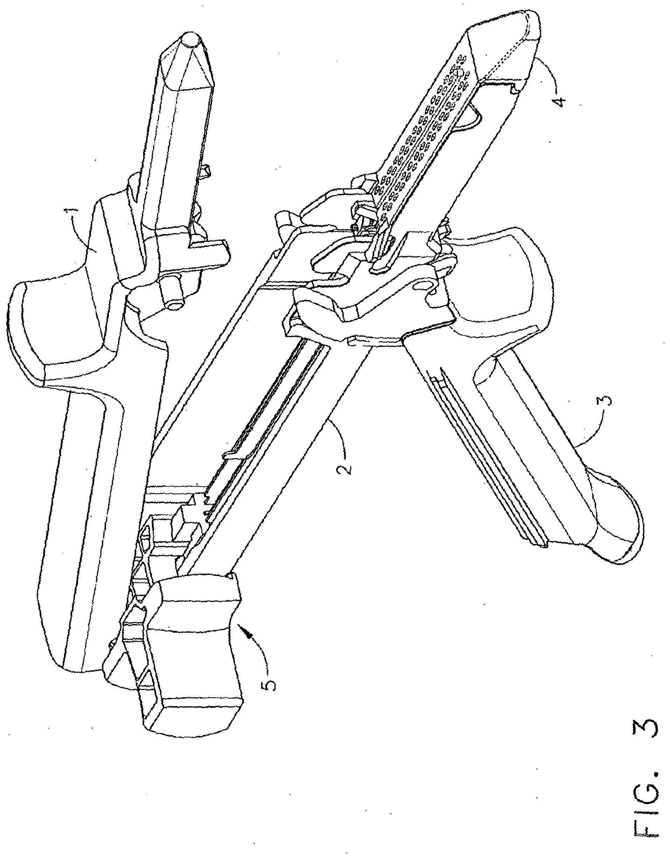

FIGURE 3 is an Isometric view of the entire device in open position.
FIGURE 4 is a Front view of the Knife module.
FIGURE 4a is a Close-up view of the module proximal end.
FIGURE 4b is a Section view of the Module proximal end showing relationship between Firing Knob and detent spring in full back position.
FIGURE 4c Section view of the Module proximal end when knob is moved forward during firing.
FIGURE 5a is an Front view of device while hook latch in ready to assemble state.
FIGURE 5b is an Front view of the device after hook latch is fully inserted state.
FIGURE 5c is an Front view of the device in rotated and locked in state.
FIGURE 5d is an Front View of device in a Pre-close position.
FIGURE 5e is an Front View of device in an open and detent position.
FIGURE 6 is an Isometric View of Reusable Anvil.
FIGURE 7 is another Isometric View of the reusable anvil.

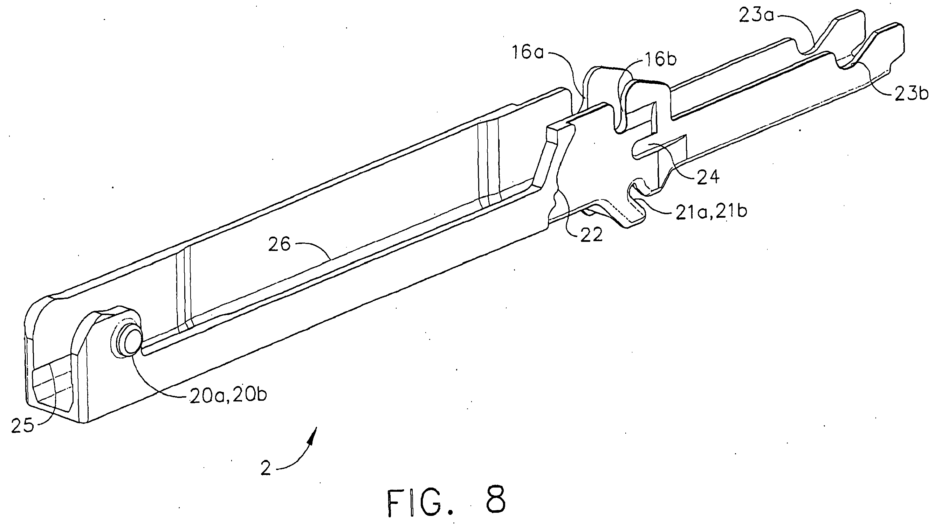

FIGURE 8 is a Isometric view of the Reusable Cartridge channel.

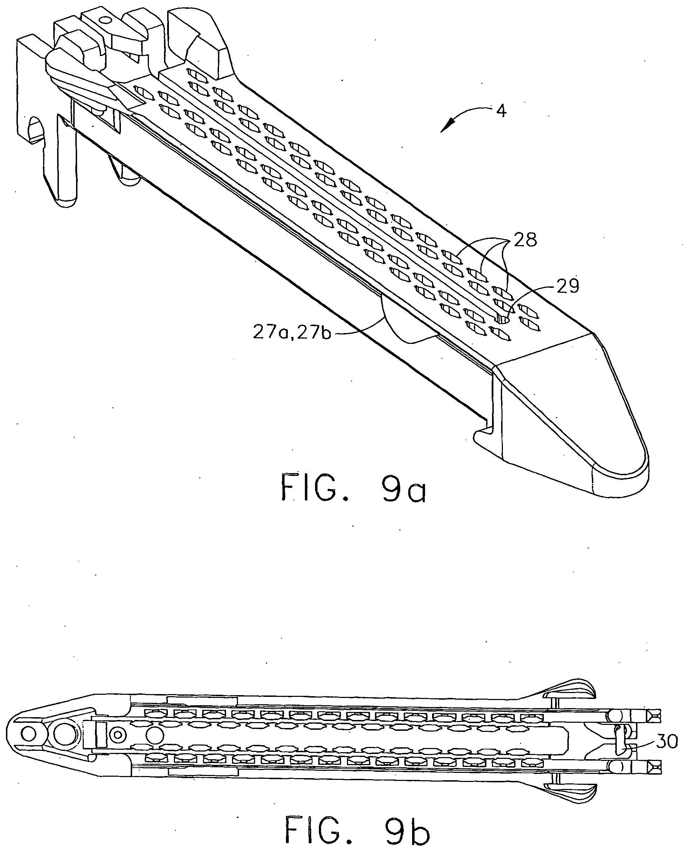

FIGURE 9a is a Isometric view of the disposable cartridge.
FIGURE 9b is a Bottom View of the disposable cartridge.

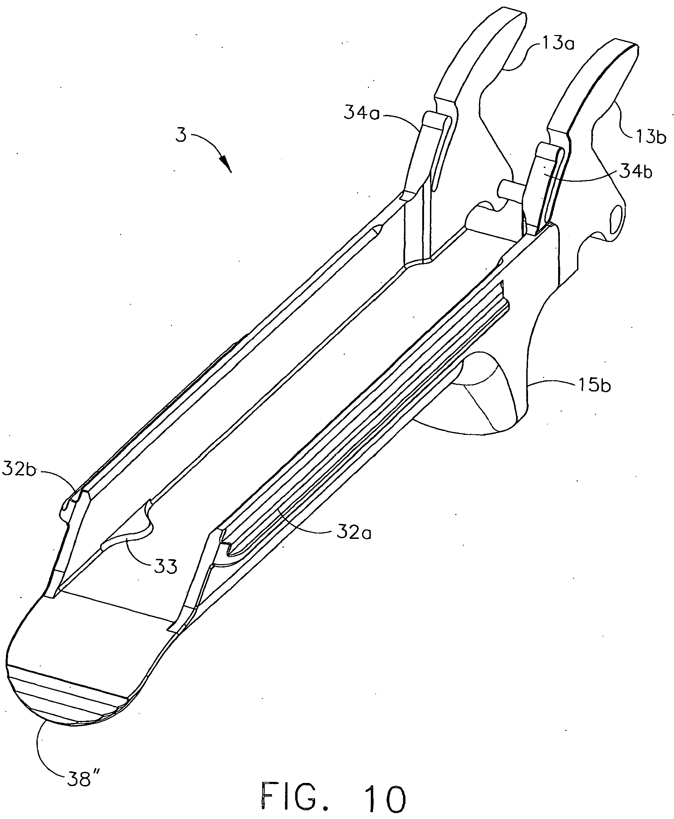

FIGURE 10 is an Isometric view of the Reusable Hook Latch.
FIGURE 11 is a Front view of the Reusable Hook Latch.

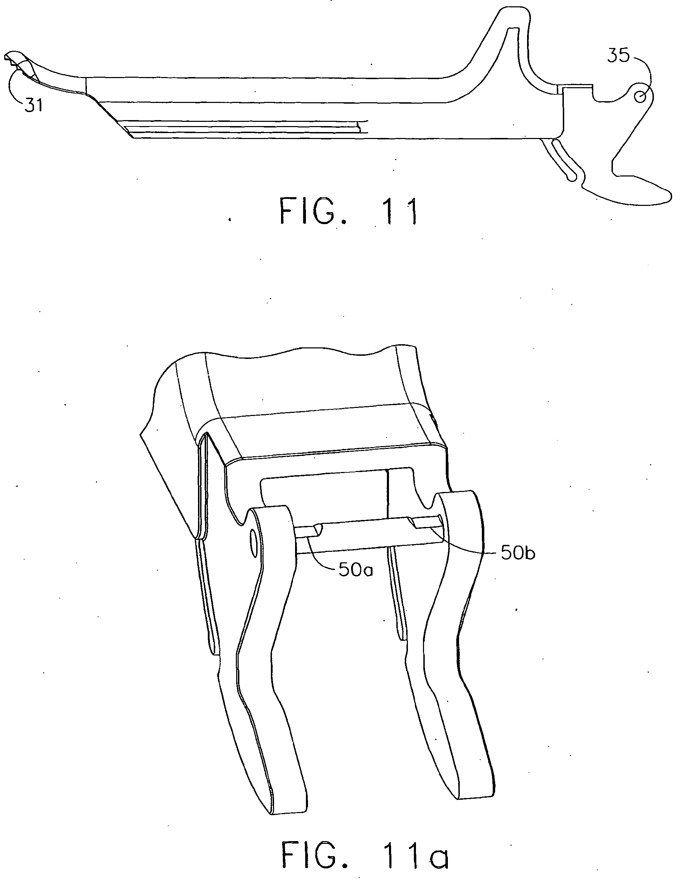

FIGURE 11 a is a Close-up view of the Hook Latch Pin showing flats.

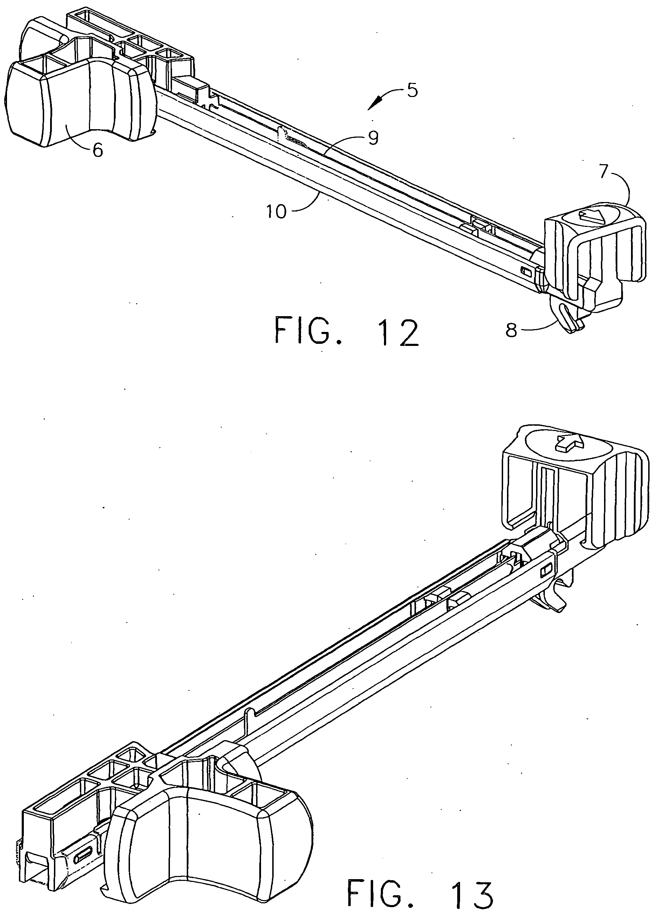

FIGURE 12 is a Isometric view of the disposable Knife Module.
FIGURE 13 is a Another Isometric view of the disposable Knife Module.

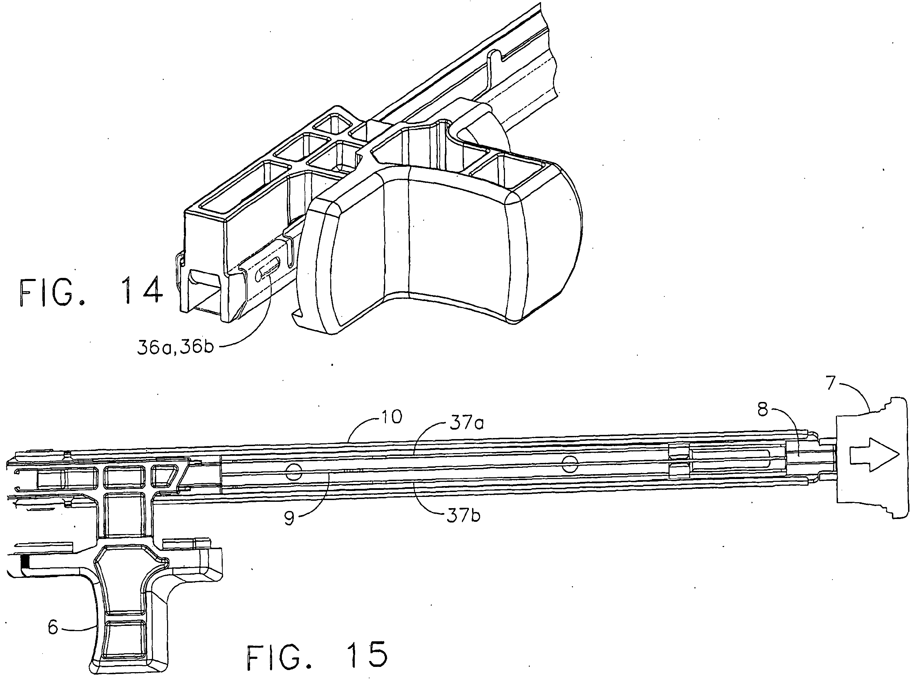

FIGURE 14 is a Close up Isometric view of the proximal end of the Module.
FIGURE 15 is a Top view of the Disposable Module.

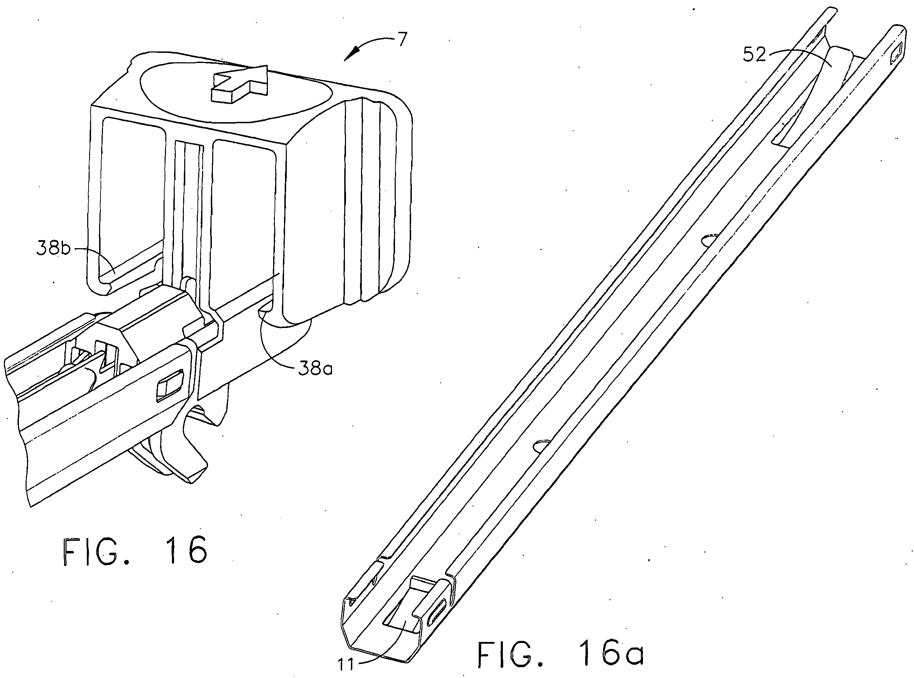

FIGURE 16 is a Close up Isometric view of the distal end of the Module.
FIGURE 16a is a Isometric view of the Module Pan.

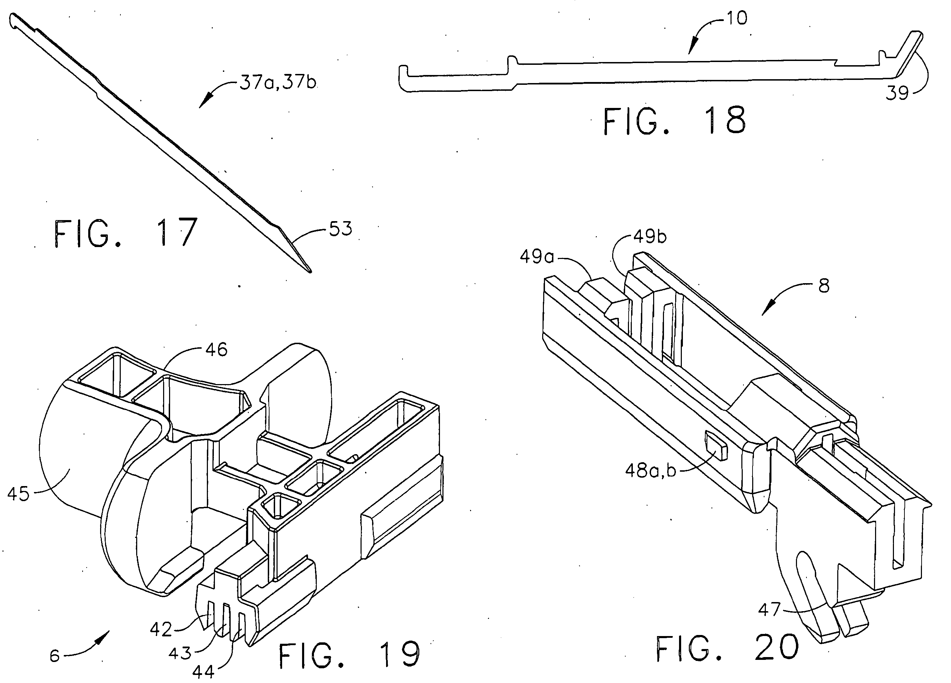

FIGURE 17 is a Isometric view of the Wedge.
FIGURE 18 is a Isometric view of the Knife.
FIGURE 19 is a Isometric view of the Firing Knob.
FIGURE 20 is a Isometric view of the Spacer.

### Detailed Description of the Invention

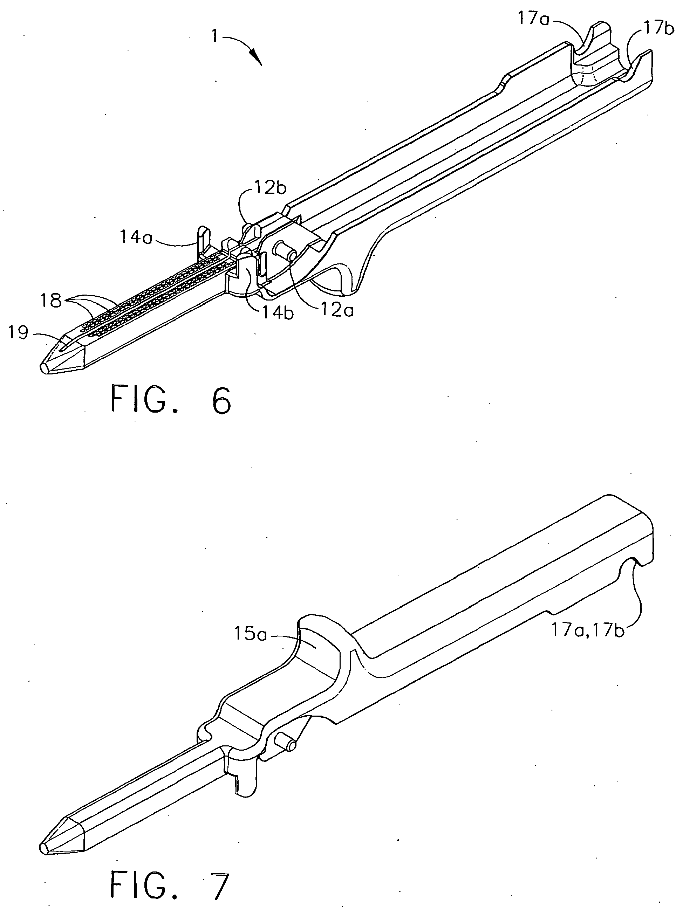

Referring now to the figures there is shown a reusable handle consisting of an Anvil (1), Cartridge Channel (2) and Hook-Latch (3). The main function of the Anvil depicted in fig 6 and fig7 is to allow the forming of B-form staples. The U shaped staples ejected from the cartridge subassembly during device firing are pushed into the staple forming pockets 18 of the Anvil resulting in a B-form. The Anvil also contains cylindrical projections 12a,12b that enable the device to be closed. These protrusions interact with the cam surfaces 13a,13b on the Hook Latch (3) to provide compression of tissue between the Anvil surface and the cartridge surface. The Anvil also contains two V shaped notches 17a, 17b which interact with projections 20 on the cartridge channel to provide a means for easily aligning the two reusable components during use. The Anvil disclosed is preferably made of single piece of metal to assist in added structural strength. Various areas could be manufactured separately and attached together with known joining methods like welding. Tissue stops 14a,14b on the Anvil are incorporated to prevent cutting of unstapled tissue.

The Cartridge Channel (2) locates and holds the single use cartridge in place. During use the notches 23a,23b on the channel are loosely engaged with projections 27a,b on the cartridge assembly. The Channel also contains alignment slots 16a,16b to allow for interface with the Anvil projections 12a,12b.The cartridge channel also contains notches 24a,24b to allow for snap connection with the protector cap 7 on Knife Module 5 through the snaps 38a, 38b. The channel also has retention features 25 at the proximal end to facilitate a snap connection with detent bumps 36a, 36b on the Module Pan 10.

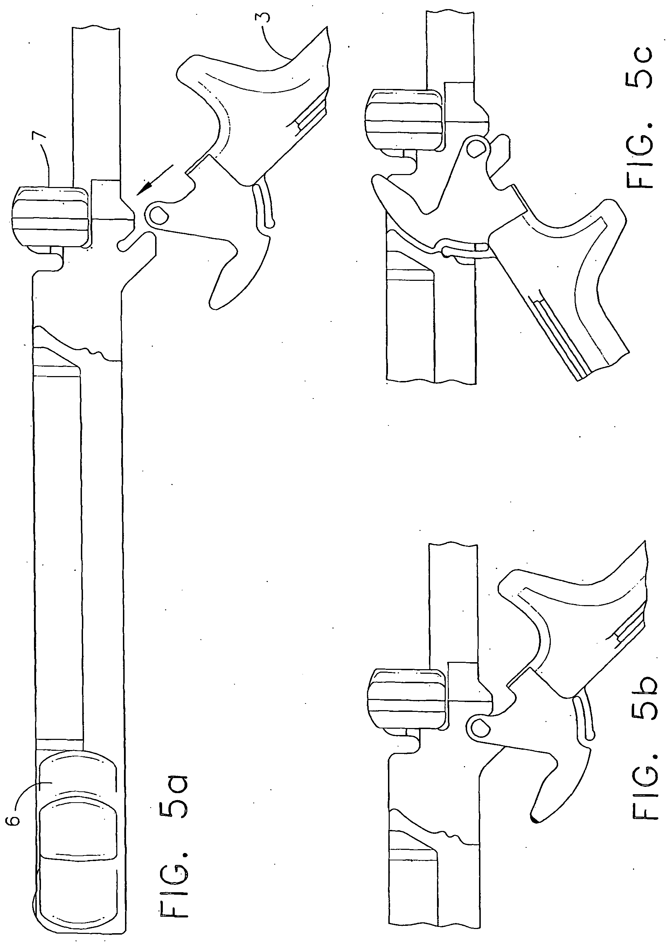

The hook latch 3 is detachably connected to the Cartridge channel via slot 31. This detachable design facilitates in easy cleaning and sterilization of the reusable components. The slot 31 has a narrow opening and ends with a circular hole slightly larger than the narrow opening. The hook latch contains a latch Pin that has two flats 50a, 50b on the generally cylindrical pin. The flats when oriented with the channel slot 31 as shown in Fig 5a allows for insertion of the hook-latch into the channel slot 31. The hook Latch can then be rotated into the operating position, in which it cannot be disengaged from the channel.

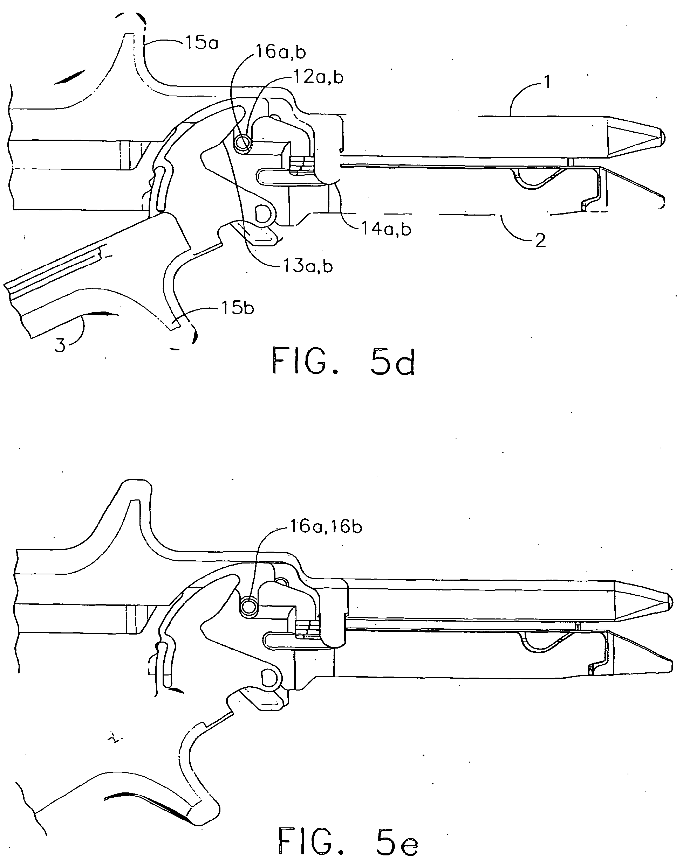

It is important to note that the Assembly position of the hook latch is different than the operating position of the Hook latch to prevent the hook latch from disengaging during operation. Fig 5a, 5b and 5c show the various positions of the hook Latch during preparation prior to use. Fig 5e shows the device in the open position during use. In this position the anvil pins 12a,12b are free to disengage from the channel slots 16a, 16b. Once tissue is positioned appropriately the device can be put in a pre-close position to allow for tissue manipulation prior to complete closure. These distinct positions are possible through the interaction between spring means 34a, 34b on the hook latch and detent bumps 22 on the channel. Fig 5d shows the pre-close position in which the Anvil pins 12a, 12b are prevented from disengaging from channel slots by the cam surfaces 13a,13b on the hook latch by obstructing the channel slots 16a, 16b.

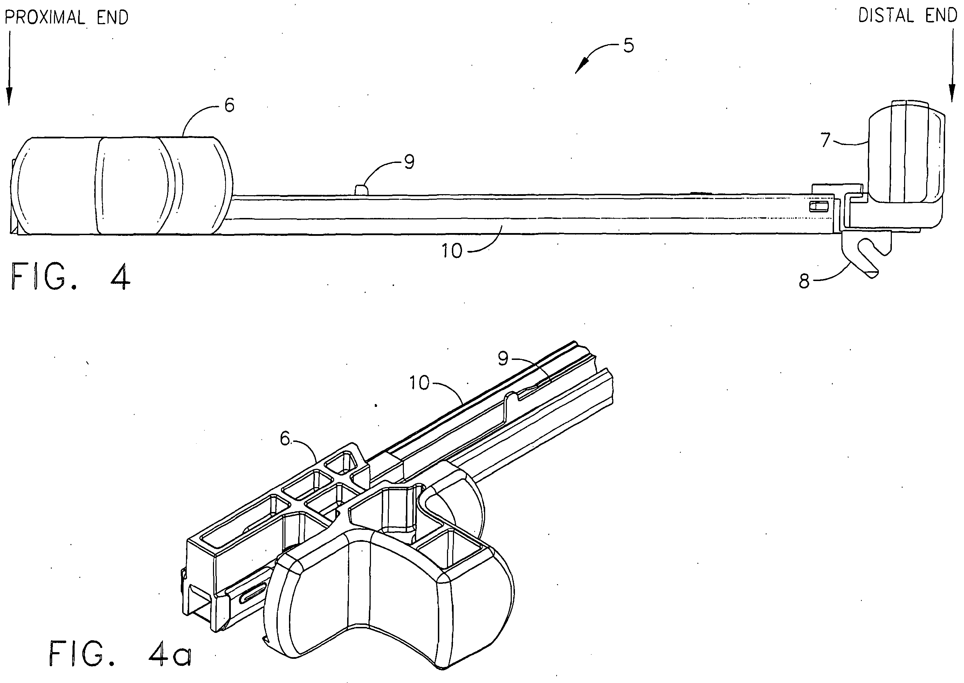

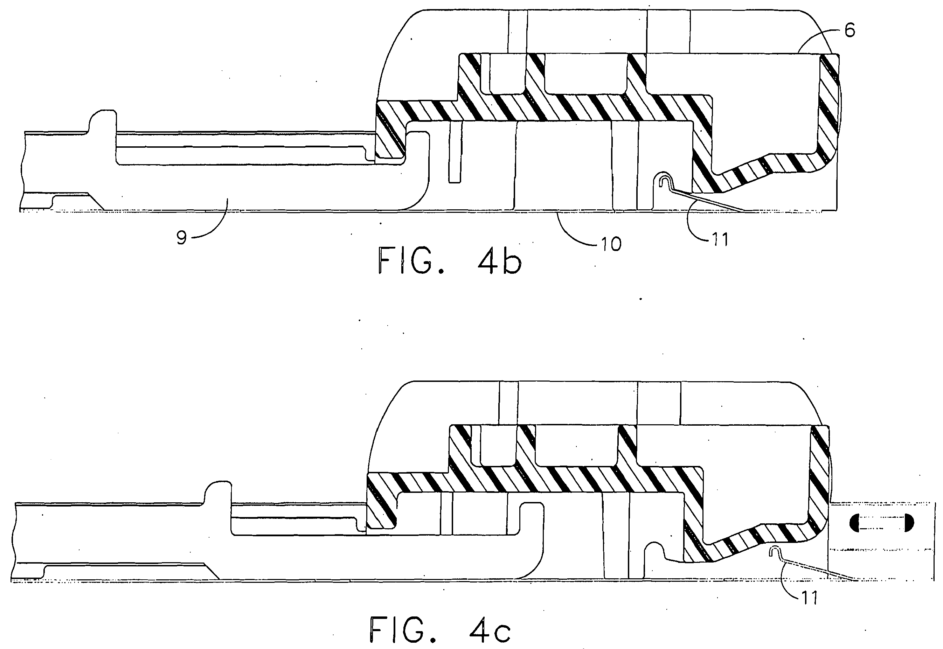

The Firing Module 5 provides the firing mechanism to allow stapling and cutting of tissue clamped in the jaws of the device. This module is preferably a single patient sterile device due to the difficulty of cleaning it. However it can be used multiple times in the same patient to reduce cost to the procedure. The main components are Firing Knob 6, Protector cap 7, Spacer 8, Knife 9, Wedges 37a, 37b, Knife 9 and Module Pan 10. Module Pan 10 is a generally U-shaped and provides the structure that supports the rest of the components together. It also contains two integrated leaf springs. Spring 52 in the distal end is used to operate a safety lockout mechanism that lifts the knife against the spacer preventing re-firing of a used cartridge. The other leaf spring 11 shown in fig 4b and 4c acts as a detent spring. Detent Spring 11 interacts with notch on the firing knob preventing forward motion of the firing knob during shipping and also provides and audible tactile feedback when the knob is fully returned. The knife and wedges are attached to and pushed by the firing knob during use. The knife is slidably constrained to the firing knob through features 40, 41 on the knife. The knife is a sheet metal component with a ground cutting edge.

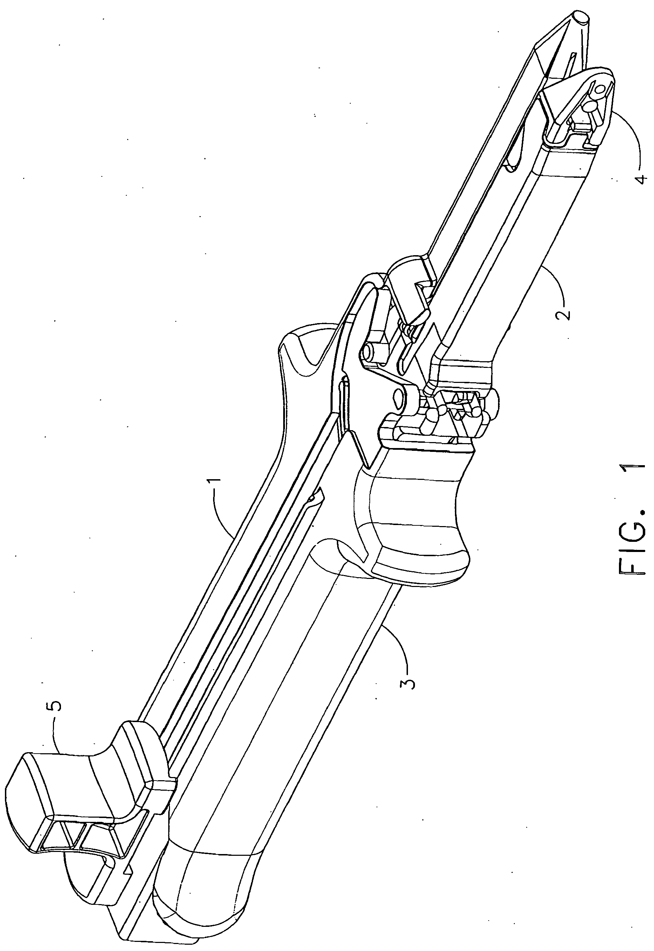

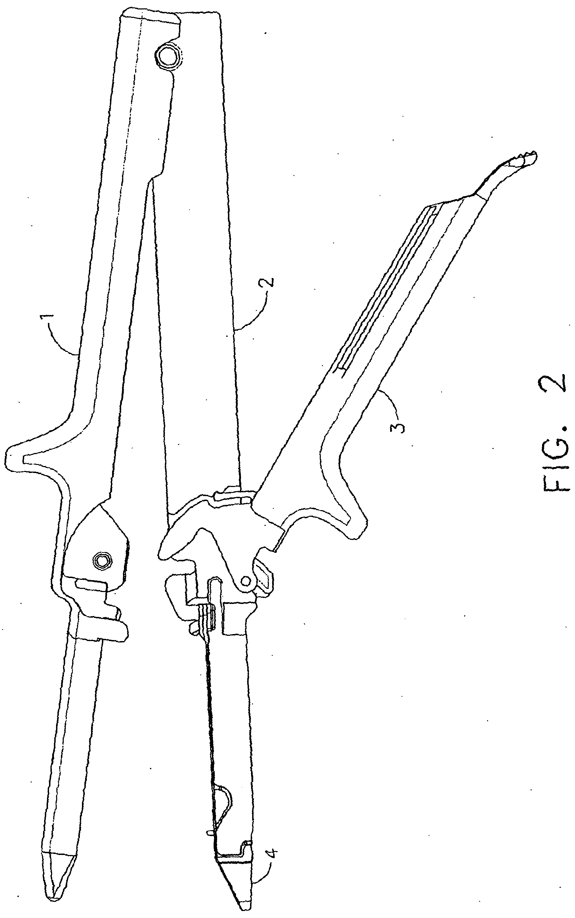

The reusable device (includes Anvil 1, cartridge channel 2, and Hook-latch 3) is provided to the user in a non-sterile package. The user has to clean the device with PH neutral enzymatic cleaners, and then steam autoclave to sterilize the device prior to each procedure. The Knife Module 5 is packaged in a sterile disposable package. The user opens the module in the sterile field. The cartridge 4 is packaged in a sterile disposable package. The user removes the cartridge from the package in the sterile field. The Knife module 5 is inserted proximal end first into the channel 2 and snapped in place. The distal end of the module is then rotated down into the channel. The protector cap 7 is pushed down to lock the Module in the channel, this holds the module in place. The hook-latch is then inserted into the channel and locked in the use position as shown in fig 5a, 5b and 5c. The protector cap is removed by moving the protector cap in the direction of the arrow embedded into the cap 7. A new cartridge is then inserted into position. Fig 2 shows the cartridge 4 in the assembled position in the channel 2. The tissue to be transected is positioned between the anvil 1 and the cartridge 4. The device is then closed by rotating the Hook Latch into the closed position shown in Fig 1. In this state the tissue is clamped in the device. The tissue stops 14a, 14b on the anvil prevent the tissue from being positioned too far into the device. The Pre-close position shown in fig 5e can used to reposition tissue prior to complete closure. In this position the Anvil and Cartridge channel are locked together by can move adequately to accommodate tissue repositioning. The device is fired by pushing the knob forward until it stops. This action staples and divides the tissue positioned in the device. The knob is returned to the full back position. The device provides a tactile feedback through the action of the detent spring on the Module Pan 10. The latch is opened to the open position. The used cartridge is removed and discarded. If required a new cartridge is inserted and device reused. Once all the required tissue is transected, the hook latch is removed by rotating to the assembly/removable position shown in Fig 5a, 5b to prepare the device for cleaning and sterilization. The knife Module is removed and discarded. The device is then cleaned and sterilized as separate components to facilitate easy cleaning. The device is then ready for reuse

While the present invention has been illustrated by description of several embodiments, it is not the intention of the applicant to restrict or limit the spirit and scope of the appended claims to such detail. Numerous variations, changes, and substitutions will occur to those skilled in the art without departing from the scope of the invention. Moreover, the structure of each element associated with the present invention can be alternatively described as a means for providing the function performed by the element. Accordingly, it is intended that the invention be limited only by the scope of the appended claims.

## Claims

1. A surgical stapler for deploying staples into tissue, said stapler comprising:
a. a body having a distal end, a proximal end and a longitudinal axis therebetween, said proximal end comprising a handle and a proximal channel, and a distal end comprising a staple cartridge channel (2) and an opposing anvil (1); and
b. an actuator module (5) for deploying staples, said actuator module (5) including at least one longitudinally moveable member (37a, 37b, 9) for sequentially ejecting staples towards said anvil (1), **characterized in that** said actuating module (5) further comprising a module pan (10) having detent means (36a, 36b) and a protector cap (7) for removably and slidably mounting said actuator module (5) within said proximal channel.

2. The surgical stapler of claim 1, further including a staple cartridge (4) containing a plurality of staples, said staple cartridge (4) being disposed within said staple cartridge channel (2).

3. The surgical stapler of claim 2, wherein said stapler body comprises an upper jaw member (1) and a lower jaw member (2) each being formed as a single piece, said upper jaw member having (1) a pair of stationary pins (12a, 12b) and said lower jaw member (2) comprising a pair of alignment slots (16a, 16b), said upper jaw pins (12a, 12b) being inserted into said lower jaw slots (16a, 16b) to align said jaw members (1, 2).

4. The surgical stapler of claim 3, wherein said stapler further comprises a latching member (3) for connecting said upper (1) and lower (2) jaw members together at an intermediate position along said longitudinal axis of said stapler, said latching member (3) being moveable relative to said upper (1) and lower (2) jaw members.

5. The surgical stapler of claim 4, wherein said latching member (3) is moveable relative to said upper (1) and lower (2) jaw members to place said stapler in a series of different locking states.

6. The surgical stapler of claim 5, wherein said latching member (3) is formed as a single piece component that can be connected to said stapler in a closed locking state and disconnected from said stapler in an open locking state.

7. The surgical stapler of claim 6, wherein said latching member (3) further comprises a latch pin (35), said latch pin (35) being insertable into and removable from a slot (16a, 16b) on said stapler body for connecting and disconnecting said latching member (3) to said stapler.

8. The surgical stapler of claim 7, wherein said stapler further comprises retaining means (22) on said stapler body for retaining said latching member (3) in said different locking states relative to said upper (1) and lower (2) jaw members.

9. The surgical stapler of claim 6, wherein said latching member (3) is a single piece component, and wherein said upper jaw member (1), said lower jaw member (2) and said latching member (3) can be disassembled from said stapler and individually reconditioned for reuse in a different patient procedure.

10. The surgical stapler of claim 8, wherein said retaining means (22) can hold said latching member in a pre-close locking state in which said upper jaw pins (12a, 12b) are prevented from disengaging from said lower jaw slots (16a, 16b) yet said staple cartridge (4) can move relative to said anvil (1).

11. The surgical stapler of claim 8, wherein said retaining means (22) can hold said latching member (3) in a partially locked in state in which said latching member (3) is connected to said lower jaw member (2), yet said upper jaw pins (12a, 12b) can disengage from said lower jaw slots (16a, 16b) to enable said upper (1) and lower (2) jaw members to be separated.

12. The surgical stapler of claim 8, wherein said latching (3) member further comprises hook latch members (13a, 13b) moveable relative to opposing sidewalls of said lower jaw member (2), said hook latch members (13a, 13b) engaging said upper jaw pins (12a, 12b) in a closed locking state to fix said upper (1) and lower (2) jaw members together in an operative position.

13. The surgical stapler of claim 7, wherein said actuator module (5) further comprises an opening (8) aligned with said stapler body opening along said longitudinal stapler axis, said latch pin (35) being insertable into and removable from said actuator module opening (8) when said pin (35) is connected to said stapler body to hold said actuator module (5) within said stapler body.

14. The surgical stapler of claim 13, further comprising structure (23a, 23b) for connecting and disconnecting staple cartridges (4) to said stapler body relative to said actuator module (5), wherein multiple staple cartridges may be used with a single actuator module (5) within said stapler body.

15. The surgical stapler of claim 1, wherein said longitudinally moveable members (37a, 37b, 9) comprise a cutting member (9) and a pair of staple actuating bars (37a, 37b), and said actuator module (5) further includes a guide means (8) for aligning said cutting member (9) and staple actuating bars (37a, 37b) with a staple cartridge (4) for reciprocal movement between said actuator module (5) and said staple cartridge (4).

## Patentansprüche

1. Chirurgisches Klammernahtgerät zum Ausbringen von Klammern in Gewebe, wobei das Klammernahtgerät Folgendes umfasst:
a. einen Körper mit einem distalen Ende, einem proximalen Ende und eine dazwischenliegende Längsachse, wobei das proximale Ende einen Griff und einen proximalen Kanal umfasst und ein distales Ende einen Klammermagazinkanal (2) und einen gegenüberliegenden Amboss (1) umfasst, und
b. ein Aktuatormodul (5) zum Ausbringen von Klammern, wobei das Aktuatormodul (5) mindestens ein in Längsrichtung bewegliches Element (37a, 37b, 9) aufweist, um Klammern nacheinander zu dem Amboss (1) hin auszustoßen, **dadurch gekennzeichnet, dass**
das Aktuatormodul (5) ferner eine Modulpfanne (10) mit Rastmitteln (36a, 36b) und einer Schutzkappe (7) zum entfernbaren und schiebbaren Montieren des Aktuatormoduls (5) in dem proximalen Kanal umfasst.

2. Chirurgisches Klammernahtgerät nach Anspruch 1, das ferner ein eine Vielzahl von Klammern enthaltendes Klammermagazin (4) aufweist, wobei das Klammermagazin (4) in dem Klammermagazinkanal (2) angeordnet ist.

3. Chirurgisches Klammernahtgerät nach Anspruch 2, wobei der Klammernahtgerätkörper ein oberes Backenelement (1) und ein unteres Backenelement (2) umfasst, die jeweils einstückig ausgebildet sind, wobei das obere Backenelement (1) ein Paar feststehende Stifte (12a, 12b) hat und das untere Backenelement (2) ein Paar Ausrichtungsschlitze (16a, 16b) umfasst, wobei die Stifte (12a, 12b) der oberen Backe zur Ausrichtung der Backenelemente (1, 2) in die Schlitze (16a, 16b) der unteren Backe eingeführt werden.

4. Chirurgisches Klammernahtgerät nach Anspruch 3, wobei das Klammernahtgerät ferner ein Riegelelement (3) umfasst, um das obere (1) und das untere (2) Backenelement an einer Zwischenposition entlang der Längsachse des Klammernahtgeräts miteinander zu verbinden, wobei das Riegelelement (3) bezüglich des oberen (1) und des unteren (2) Backenelements beweglich ist.

5. Chirurgisches Klammernahtgerät nach Anspruch 4, wobei das Riegelelement (3) bezüglich des oberen (1) und des unteren (2) Backenelements beweglich ist, um das Klammernahtgerät in eine Reihe von unterschiedlichen Verriegelungszuständen zu platzieren.

6. Chirurgisches Klammernahtgerät nach Anspruch 5, wobei das Riegelelement (3) als eine einstückige Komponente ausgebildet ist, die in einem geschlossenen Verriegelungszustand mit dem Klammernahtgerät verbunden und in einem offenen Verriegelungszustand von dem Klammernahtgerät getrennt werden kann.

7. Chirurgisches Klammernahtgerät nach Anspruch 6, wobei das Riegelelement (3) ferner einen Riegelstift (35) umfasst, wobei der Riegelstift (35) in einen Schlitz (16a, 16b) an dem Klammernahtgerätkörper eingeführt und daraus entfernt werden kann, um das Riegelelement (3) mit dem Klammernahtgerät zu verbinden und davon zu trennen.

8. Chirurgisches Klammernahtgerät nach Anspruch 7, wobei das Klammernahtgerät ferner ein Haltemittel (22) an dem Klammernahtgerätkörper umfasst, um das Riegelelement (3) in den unterschiedlichen Verriegelungszuständen bezüglich des oberen (1) und des unteren (2) Backenelements zu halten.

9. Chirurgisches Klammernahtgerät nach Anspruch 6, wobei das Riegelelement (3) eine einstückige Komponente ist und wobei das obere Backenelement (1), das untere Backenelement (2) und das Riegelelement (3) von dem Klammernahtgerät abgebaut und einzeln für die Wiederverwendung in einem anderen Patientenverfahren aufbereitet werden können.

10. Chirurgisches Klammernahtgerät nach Anspruch 8, wobei das Haltemittel (22) das Riegelelement in einem Vorverschluss-Verriegelungszustand halten kann, in dem die Stifte (12a, 12b) der oberen Backe daran gehindert werden, aus den Schlitzen (16a, 16b) der unteren Backe auszurücken, sich das Klammermagazin (4) aber bezüglich des Ambosses (1) bewegen kann.

11. Chirurgisches Klammernahtgerät nach Anspruch 8, wobei das Haltemittel (22) das Riegelelement (3) in einem teilweise verriegelten Zustand halten kann, in dem das Riegelelement (3) mit dem unteren Backenelement (2) verbunden ist, die Stifte (12a, 12b) der oberen Backe aber aus den Schlitzen (16a, 16b) der unteren Backe ausrücken können, damit das obere (1) und das untere (2) Backenelement getrennt werden können.

12. Chirurgisches Klammernahtgerät nach Anspruch 8, wobei das Riegelelement (3) ferner Hakenriegelelemente (13a, 13b) umfasst, die bezüglich gegenüberliegenden Seitenwänden des unteren Backenelements (2) beweglich sind, wobei die Hakenriegelelemente (13a, 13b) die Stifte (12a, 12b) der oberen Backe in einem geschlossenen Verriegelungszustand in Eingriff nehmen, um das obere (1) und das untere (2) Backenelement in einer Betriebsposition aneinander zu befestigen.

13. Chirurgisches Klammernahtgerät nach Anspruch 7, wobei das Aktuatormodul (5) ferner eine Öffnung (8) umfasst, die auf den Klammernahtgerätkörper ausgerichtet und entlang der Klammernahtgerätlängsachse offen ist, wobei der Riegelstift (35) in die Aktuatormodulöffnung (8) eingeführt und daraus entfernt werden kann, wenn der Stift (35) mit dem Klammernahtgerätkörper verbunden ist, um das Aktuatormodul (5) in dem Klammernahtgerätkörper zu halten.

14. Chirurgisches Klammernahtgerät nach Anspruch 13, ferner umfassend eine Struktur (23a, 23b), um Klammermagazine (4) mit dem Klammernahtgerätkörper bezüglich des Aktuatormoduls (5) zu verbinden und davon zu trennen, wobei mehrere Klammermagazine mit einem einzigen Aktuatormodul (5) in dem Klammernahtgerätkörper verwendet werden können.

15. Chirurgisches Klammernahtgerät nach Anspruch 1, wobei die in Längsrichtung beweglichen Elemente (37a, 37b, 9) ein Schneidelement (9) und ein Paar Klammerbetätigungsstäbe (37a, 37b) umfassen und das Aktuatormodul (5) ferner ein Führungsmittel (8) aufweist, um das Schneidelement (9) und die Klammerbetätigungsstäbe (37a, 37b) zur hin- und hergehenden Bewegung zwischen dem Aktuatormodul (5) und dem Klammermagazin (4) auf das Klammermagazin (4) auszurichten.

## Revendications

1. Agrafeuse chirurgicale pour mettre en oeuvre des agrafes dans un tissu, ladite agrafeuse comprenant :
a. un corps ayant une extrémité distale, une extrémité proximale et un axe longitudinal entre celles-ci, ladite extrémité proximale comprenant une poignée et un canal proximal et une extrémité distale comprenant un canal (2) de cartouche à agrafes et une enclume (1) en face ; et
b. un module d'actionneur (5) pour mettre en oeuvre des agrafes, ledit module d'actionneur (5) comprenant au moins un élément (37a, 37b, 9) mobile longitudinalement pour éjecter séquentiellement les agrafes vers ladite enclume (1),
**caractérisée en ce que** ledit module d'actionnement (5) comprend en outre un plateau (10) de module comportant des moyens d'encliquetage (36a, 36b) et un capuchon de protection (7) pour monter de façon amovible et coulissante ledit module d'actionneur (5) à l'intérieur dudit canal proximal.

2. Agrafeuse chirurgicale selon la revendication 1, comprenant en outre une cartouche (4) à agrafes contenant une pluralité d'agrafes, ladite cartouche (4) à agrafes étant disposée à l'intérieur dudit canal (2) de cartouche à agrafes.

3. Agrafeuse chirurgicale selon la revendication 2, dans lequel ledit corps d'agrafeuse comprend un élément de mâchoire supérieure (1) et un élément de mâchoire inférieure (2), chacune étant formée d'une seule pièce, ledit élément de mâchoire supérieure (1) comportant une paire d'axes fixes (12a, 12b) et ledit élément de mâchoire inférieure (2) comprenant une paire de fentes d'alignement (16a, 16b), lesdits axes (12a, 12b) de la mâchoire supérieure étant insérés dans lesdites fentes (16a, 16b) de la mâchoire inférieure pour aligner lesdits éléments de mâchoire (1, 2).

4. Agrafeuse chirurgicale selon la revendication 3, comprenant en outre un élément de verrouillage (3) pour raccorder ensemble lesdits éléments de mâchoire supérieur (1) et inférieur (2) au niveau d'une position intermédiaire le long dudit axe longitudinal de ladite agrafeuse, ledit élément de verrouillage (3) étant mobile par rapport auxdits éléments de mâchoire supérieur (1) et inférieur (2).

5. Agrafeuse chirurgicale selon la revendication 4, dans laquelle ledit élément de verrouillage (3) est mobile par rapport auxdits éléments de mâchoire supérieur (1) et inférieur (2) pour placer ladite agrafeuse dans une série d'états de verrouillage différents.

6. Agrafeuse chirurgicale selon la revendication 5, dans laquelle ledit élément de verrouillage (3) est constitué en tant que composant monobloc qui peut être raccordé à ladite agrafeuse dans un état de verrouillage fermé et séparé de ladite agrafeuse dans un état de verrouillage ouvert.

7. Agrafeuse chirurgicale selon la revendication 6, dans laquelle ledit élément de verrouillage (3) comprend en outre un axe de verrouillage (35), ledit axe de verrouillage (35) pouvant être inséré dans une fente (16a, 16b) sur ledit corps d'agrafeuse et en être retiré pour raccorder ledit élément de verrouillage (3) à ladite agrafeuse et l'en séparer.

8. Agrafeuse chirurgicale selon la revendication 7, comprenant en outre des moyens de retenue (22) sur ledit corps d'agrafeuse pour retenir ledit élément de verrouillage (3) dans lesdits différents états de verrouillage par rapport par rapport auxdits éléments de mâchoire supérieur (1) et inférieur (2).

9. Agrafeuse chirurgicale selon la revendication 6, dans laquelle ledit élément de verrouillage (3) est un composant monobloc, et dans laquelle ledit élément de mâchoire supérieur (1), ledit élément de mâchoire inférieur (2) et ledit élément de verrouillage (3) peuvent être démontés de ladite agrafeuse et remis individuellement en état pour être réutilisés dans le traitement d'un patient différent.

10. Agrafeuse chirurgicale selon la revendication 8, dans laquelle ledit moyen de retenue (22) peut maintenir ledit élément de verrouillage dans un état de verrouillage avant fermeture dans lequel lesdits axes (12a, 12b) de la mâchoire supérieure sont empêchés de se dégager desdites fentes (16a, 16b) de la mâchoire inférieure et cependant ladite cartouche (4) à agrafes peut se déplacer par rapport à ladite enclume (1).

11. Agrafeuse chirurgicale selon la revendication 8, dans laquelle ledit moyen de retenue (22) peut maintenir ledit élément de verrouillage (3) dans un état partiellement verrouillé dans lequel ledit élément de verrouillage (3) est raccordé audit élément de mâchoire inférieur (2) et cependant lesdits axes (12a, 12b) de la mâchoire supérieure peuvent se dégager desdites fentes (16a, 16b) de la mâchoire inférieure pour permettre auxdits éléments de mâchoire supérieur (1) et inférieur (2) de se séparer.

12. Agrafeuse chirurgicale selon la revendication 8, dans laquelle ledit élément de verrouillage (3) comprend en outre des éléments de verrouillage par crochet (13a, 13b) mobiles par rapport aux parois latérales opposées dudit élément de mâchoire inférieure (2), lesdits éléments de verrouillage par crochet (13a, 13b) entrant en prise avec lesdits axes (12a, 12b) de la mâchoire supérieure dans un état de verrouillage fermé pour fixer ensemble lesdits éléments de mâchoire supérieur (1) et inférieur (2) dans une position opératoire.

13. Agrafeuse chirurgicale selon la revendication 7, dans laquelle ledit module d'actionneur (5) comprend en outre une ouverture (8) alignée avec ledit corps d'agrafeuse et s'ouvrant suivant ledit axe longitudinal d'agrafeuse, ledit axe de verrouillage (35) pouvant être inséré dans ladite ouverture (8) du module d'actionneur et retiré de celle-ci quand ledit axe (35) est raccordé audit corps d'agrafeuse pour maintenir ledit module d'actionneur (5) à l'intérieur dudit corps d'agrafeuse.

14. Agrafeuse chirurgicale selon la revendication 13, comprenant en outre une structure (23a, 23b) pour raccorder les cartouches (4) d'agrafes audit corps d'agrafeuse et les en séparer relativement audit module d'actionneur (5), dans laquelle on peut utiliser de multiples cartouches d'agrafes avec un seul module d'actionneur (5) à l'intérieur dudit corps d'agrafeuse.

15. Agrafeuse chirurgicale selon la revendication 1, dans laquelle lesdits éléments (37a, 37b, 9) mobiles longitudinalement comprennent un élément de coupe (9) et une paire de barres d'actionnement (37a, 37b) des agrafes et ledit module d'actionneur (5) comprend en outre un moyen de guidage (8) pour aligner ledit élément de coupe (9) et lesdites barres d'actionnement (37a, 37b) des agrafes avec une cartouche (4) d'agrafes pour un mouvement de va-et-vient entre ledit module d'actionneur (5) et ladite cartouche (4) d'agrafes.
